# EUROPEAN PATENT SPECIFICATION

(11) **EP 3 717 067 B1**
(45) Date of publication and mention of the grant of the patent: **11.10.2023**
(21) Application number: 18829562.0
(22) Date of filing: 30.11.2018
(51) Int. Cl.: A61N 5/06

(54) **SYSTEM FOR LONG-TERM MODULATION OF PERIPHERAL NERVE ACTIVITY WITH LIGHT**
SYSTEM ZUR LANGZEITMODULATION DER AKTIVITÄT VON PERIPHEREN NERVEN MIT LICHT
SYSTÈME DE MODULATION À LONG TERME DE L'ACTIVITÉ DU NERF PÉRIPHÉRIQUE AVEC DE LA LUMIÈRE

(30) Priority: 30.11.2017 US 201762592623 P
(43) Date of publication of application: 07.10.2020
(73) Proprietor: Case Western Reserve University, Cleveland, OH 44106 (US); Vanderbilt University, Nashville, TN 37240 (US)
(72) Inventor: JENKINS, Michael W., Cleveland Ohio 44106 (US); LEWIS, Stephen J., Cleveland Ohio 44106 (US); CHIEL, Hillel J., Cleveland Ohio 44106 (US); JANSEN, Eric Duco, Cleveland Ohio 44106 (US); ZHOU, Junqi, Cleveland Ohio 44106 (US); LOTHET, Emilie, Cleveland OH 44106 (US); KOLA, Gjinovefa, Cleveland Ohio 44106 (US)
(74) Representative: Wilson Gunn
(86) International application number: PCT/US2018/063417
(87) International publication number: WO 2019/109002

(56) References cited:
- US-A1- 2011 295 331
- US-A1- 2013 237 906
- US-A1- 2017 080 244
- US-B1- 8 996 131

## Description

### Related Application

This application claims priority to U.S. Provisional Application Serial No. 62/592,623, filed November 30, 2017, entitled "INFRARED MODULATION".

### Technical Field

The present disclosure relates generally to neural modulation and, more specifically, to systems and methods for using light for long-term modulation of peripheral nerve activity.

### Background

Disorders of the brainstem, spinal cord (C3-C5), and one or more peripheral nerves can negatively disrupt muscle activity related to respiration. Traditional solutions to this disruption of respiration include mechanical ventilation and diaphragmatic pacing, both leading to serious physiological consequences and high cost. Recently, intermittent hypoxia (IH) has become another option for treating disrupted respiration, involving short exposures to hypoxia to increase the motor output to respiratory muscles. However, IH can lead to physiological consequences, including hippocampal cell death, hypertension, and inflammation.

### Summary

The present invention provides a system according to claim 1. Further aspects and preferred embodiments are defined in the dependent claims. Aspects, embodiments, examples, and methods of the present disclosure that do not fall within the scope of the appended claims do not form part of the invention and are merely provided for illustrative purposes.

Neural modulation with light can provide another option for treating disrupted respiration, as well as other biological responses. The present disclosure relates to systems and methods for applying light, which can lead to long-term modulation of this peripheral nerve activity.

In an exemplary aspect, the present disclosure can include a method for long-term modulation of peripheral nerve activity. The method can include applying light to the peripheral nerve. The illumination can be configured with an intensity for a time to achieve a target biological response. The configured illumination can be delivered to the peripheral nerve by an optrode. The target biological response can be exhibited, as long-term modulation, for a period after the time the light is applied to the peripheral nerve.

In another aspect, the present disclosure can include a system that can be used to provide long-term modulation of peripheral nerve activity. The system can include a controller, a light generator, and an optrode. The controller can be configured to illuminate with an intensity and a time of application to achieve a target biological effect. The generator can be configured to receive the configuration from the controller and generate the light according to the configuration (intensity and time). The optrode can be configured to be placed proximal to at least a portion of a peripheral nerve to apply the light with the intensity and for the time of application to achieve the target biological effect. The target biological effect can extend for a period of time beyond the time of application of the light.

### Brief Description of the Drawings

The foregoing and other features of the present disclosure will become apparent to those skilled in the art to which the present disclosure relates upon reading the following description with reference to the accompanying drawings, in which:
FIG. 1 is a diagram showing a system that can use a light signal for long-term modulation of peripheral nerve activity in accordance with an aspect of the present disclosure;
FIG. 2 is a diagram showing an example configuration of the controller in FIG. 1;

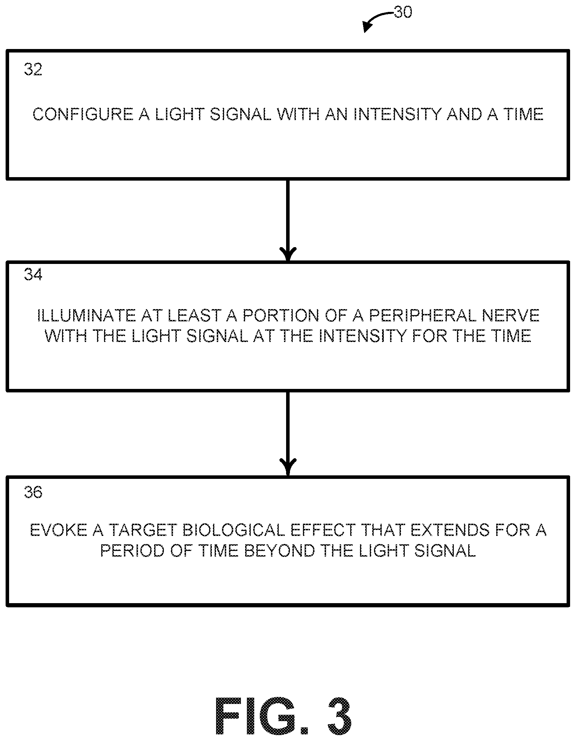

FIG. 3 is a process flow diagram illustrating a method using light for long-term modulation of peripheral nerve activity according to another aspect of the present disclosure;
FIG. 4 shows an example experimental configuration;
FIG. 5 shows how application of infrared (IR) light increases the amplitude of conduction in the phrenic nerve;
FIG. 6 shows how application of IR light increases the amplitude of conduction in the phrenic nerve without affecting breathing;
FIGS. 7 and 8 show how brief IR stimulation evokes long term effects; and
FIG. 9 shows how a serotonin blocker diminished the effect of subsequent IR application.

### Detailed Description

### I. Definitions

Unless otherwise defined, all technical terms used herein have the same meaning as commonly understood by one of ordinary skill in the art to which the present disclosure pertains.

As used herein, the singular forms "a," "an" and "the" can also include the plural forms, unless the context clearly indicates otherwise.

As used herein, the terms "comprises" and/or "comprising," can specify the presence of stated features, steps, operations, elements, and/or components, but do not preclude the presence or addition of one or more other features, steps, operations, elements, components, and/or groups.

As used herein, the term "and/or" can include any and all combinations of one or more of the associated listed items.

As used herein, the terms "first," "second," *etc.* should not limit the elements being described by these terms. These terms are only used to distinguish one element from another. Thus, a "first" element discussed below could also be termed a "second" element without departing from the teachings of the present disclosure. The sequence of operations (or acts/steps) is not limited to the order presented in the claims or figures unless specifically indicated otherwise.

As used herein, the term "neural modulation" or "neuromodulation" can refer to the alteration of nerve activity through targeted delivery of a stimulus to one or more specific neurological sites in a patient's body. The targeted delivery of the stimulus can be used for stimulation or inhibition of nerve activity.

As used herein, the term "conduction" can refer to the transmission of one or more action potentials.

As used herein, the term "inhibition" can refer to interference, with or restraint of, conduction. In other words, inhibition can be used to impede the transmission of action potentials.

As used herein, the term "threshold" can refer to a maximum or minimum change in intensity resulting in a functional result. For example, the functional result can be enhancing conduction or inhibiting conduction caused by application of a certain amount of stimulus.

As used herein, the term "light signal" (or, equivalently, "light") can refer to any signal including electromagnetic radiation that can be used as a stimulus. On example of a light signal can be an infrared (IR) light signal.

As used herein, the term "intensity" can refer to a magnitude of a property (radiant exposure per unit time) of a light signal.

As used herein, the term "radiant exposure" can refer to an amount of radiant energy received by a surface per unit area (expressed in J/cm²/pulse). Equivalently, the radiant exposure can be expressed as the irradiance that reaches a surface area due to irradiance, maintained for a particular duration of time.

As used herein, the term "target area" can refer to an area of a patient's nervous system selected to receive a stimulus for neuromodulation. In some instances, the target area can be at least a portion of a peripheral nerve chosen based on a desired target biological response.

As used herein, the terms "long-term modulation" and "long-term facilitation" can refer to a target biological response related to application of light continuing after the application of the light.

As used herein, the term "proximal" can refer to something being positioned close to something else. Although the term proximal may also encompass being on or within, something proximal need not be on or within.

As used herein, the term "subject" can refer to any warm-blooded organism including, but not limited to, a human being, a pig, a rat, a mouse, a bird, a dog, a cat, a goat, a sheep, a horse, a monkey, an ape, a rabbit, a cow, etc. The term "patient" can be used interchangeably with "subject".

### II. Overview

Disorders of the nervous system can negatively disrupt muscle activity related to one or more biological responses. For example, a disorder can be related to a phrenic nerve, and the biological response can be related to movement of a diaphragm innervated by the phrenic nerve. As another example, the disorder can be related to a hypoglossal nerve and the biological response can be related to movement of a tongue innervated by the hypoglossal nerve. As a further example, the disorder can be related to an intercostal nerve and the biological response can be related to movement of an intercostal muscle innervated by the hypoglossal nerve. As another example, the peripheral nerve can be a spinal motor neuron and the biological response can be related to movement of a muscle innervated by the spinal motor neuron. Traditional solutions to these disorders involve serious physiological consequences and/or high cost.

Neural modulation with light (like an infrared (IR) light signal) can provide another option for treating negatively disrupt muscle activity related to one or more biological responses without the negative physiological consequences common with traditional treatments. Advantageously, application of light leads to long-term modulation of this peripheral nerve activity. While not wishing to be bound by theory, it appears that application of the light works similarly to intermittent hypoxia (IH) affecting the neurotransmitter serotonin, thereby increasing the amplitude of conduction of the peripheral nerves. In addition to the long-term modulation, neural modulation with light signals provide a high spatial selectivity, even for axonal sub-populations within a nerve, and level of precision not seen with traditional electrical and magnetic stimuli. The light does not provide any stimulation artifacts, requires no exogenous agents to be added to tissue, and is not affected by external magnetic fields. Moreover, optrodes delivering the light need only be proximal to the peripheral nerve and are not required to contact the peripheral nerve.

### III. Systems

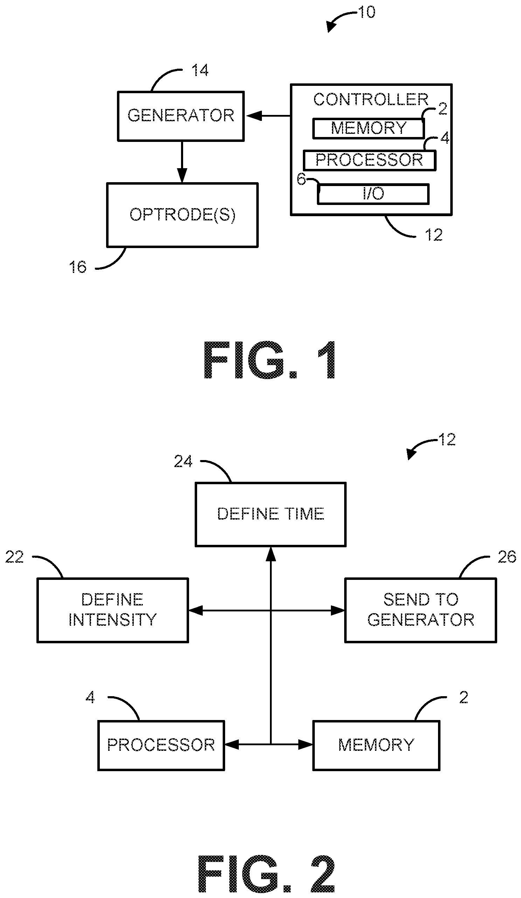

One aspect of the present disclosure can include a system 10 (FIG. 1) that can use a light signal for long-term modulation of peripheral nerve activity. The light can be applied for a short time, while a biological effect due to the application can extend for a longer period after the application of the light. For example, the light can be applied for one minute or less and the biological effect can extend for ten minutes or more. As another example, the light can be applied for 30 seconds or less and the biological effect can extend for two minutes or more.

In addition to the long-term modulation, another significant benefit of using a light signal (e.g., IR light) compared to other neuromodulatory stimuli (e.g., an electrical signal or a magnetic signal) is spatial selectivity and precision of stimulation. For example, the light can be used to select a certain axonal sub-population within the peripheral nerve for modulating (with stimulation and/or block). Additionally, stimulation with light does not require contact with the at least the portion of the target nerve for stimulation (although the light signal can be delivered directly to the target nerve, the light need only be delivered proximal to the target nerve), stimulation with light does not create stimulation artifacts, and stimulation with the light does not require exogenous agents to be added to the tissue being stimulated. Additionally, the light is not affected by magnetic field.

The system 10 can include a controller 12, a generator 14, and one or more optrodes 16. The controller 12 can be coupled to the generator 14, which can be coupled to the one or more optrodes 16. In some instances, the coupling between the controller 12 and the generator 14 and/or the coupling between the generator 14 and the one or more optrodes 16 can be via a wired connection. In other instances, the coupling between the controller 12 and the generator 14 and/or the coupling between the generator 14 and the one or more optrodes 16 can be via a wireless connection. In still other instances, the coupling between the controller 12 and the generator 14 and/or the coupling between the generator 14 and the one or more optrodes 16 can be via a connection that is both wired and wireless. In some instances, at least a portion of the controller 12 and at least a portion of the generator 14 can be included within a single hardware device, but in other instances, the controller 12 and the generator 14 can be entirely distinct hardware devices. Additionally, each element of the system 10 may have additional components to aid in the coupling that are not illustrated.

The controller 12 can be configured to define a configuration of a light signal (e.g., with certain parameters based on the intended biological effect). The parameters can include at least an intensity and a time (in other words, an amount of radiant exposure to be delivered for a time). In response to the radiant exposure being applied to at least a portion of a peripheral nerve, a certain biological response can be evoked and can extend (or carry over) after the application of the light signal.

The controller 12 can include at least a non-transitory memory 2, a processor 4, and an input/output (I/O) 6. The non-transitory memory 2 can store machine executable instructions, which are executable by the processor 4 (for example, as shown in FIG. 2, the machine executable instructions can include define an intensity of the stimulation to be delivered by the light 22, define a time for application of the light 24, and send the configuration to the generator 26). In some instances, the non-transitory memory 2 and the processor 4 can be combined in a single hardware element (e.g., a microprocessor), but in other instances, the non-transitory memory 2 and the processor 4 can include at least partially distinct hardware elements. The I/O 6 can provide an output that includes the configuration of the signal to the generator 14. In some instances, the I/O 6 can receive an input (e.g., based on a manual input, based on a predefined prescription, based on a property recorded by one or more sensors, or the like) that aids in the configuration of the light.

The generator 14 can be configured to receive the output from the controller 12 and generate the light with the specified configuration. The generator 14 can be any device configured or programmed to generate the specified illumination for application to the certain portion of the nerve. For example, when using an IR light signal that includes one or more pulses of IR, the generator 14 can be an IR light source, like at least one IR laser and/or at least one IR emitting diode. It will be appreciated that the generator 14 can include additional components to selectively configure the illumination, such as a modulator (not shown).

The generator 14 can deliver the light to the one or more optrodes 16 for application to the certain portion of the patient's nerve. For example, the generator 14 can be connected to the one or more optrodes 16 by a fiber optic cable (e.g., a flexible fiber optic cable made at least in part of a polymer material) to facilitate delivery of the light from the generator 14 to the one or more optrodes 16. The one or more optrodes 16 can be placed proximal to one or more portions of the peripheral nerve and/or different peripheral nerves.

In some instances, the one or more optrodes 16 can contact the portion of the peripheral nerve (e.g., within a nerve cuff device surrounding at least the portion of the peripheral nerve). In other instances, the one or more optrodes 16 can be located close to the peripheral nerve without contacting the peripheral nerve (e.g., within a nerve cuff device surrounding at least the portion of the peripheral nerve). The one or more optrodes 16 can deliver the light signal to the at least the portion of the peripheral nerve...

### IV. Methods

Another exemplary aspect of the present disclosure can include a method 30 for using light for long-term modulation of peripheral nerve activity. The method 30 can be executed using at least a portion of the system 10 shown in FIGS. 1 and 2 and described above. The method 30 is illustrated as a process flow diagram with flowchart illustrations. For purposes of simplicity, the method 30 is shown and described as being executed serially; however, it is to be understood and appreciated that the present disclosure is not limited by the illustrated order as some steps could occur in different orders and/or concurrently with other steps shown and described herein. Moreover, not all illustrated aspects may be required to implement the method 30.

At step 32, the light can be configured (e.g., by controller 12) with an intensity and a time of application. For example, the light can be configured to deliver an amount of radiant exposure to at least a portion of a peripheral nerve for the time. For example, the time of application can be one minute or less. As another example, the time of application can be thirty seconds or less.

At step 34, at least a portion of a peripheral nerve can be illuminated (e.g., stimulated through one or more optrodes 16) with the light signal (generated by the generator 14) at the intensity for the time. For example, an infrared (IR) light signal (e.g., including one or more pulses of IR light) can be used and delivered by an IR generator, such as one or more IR lasers or one or more IR emitting diodes, and delivered to one or more optrodes through a flexible fiber optic (e.g., including a polymer material). Application of the light can trigger a biological effect.

At step 36, the biological effect can extend (or carry over) for a period of time beyond the illumination time (in other words, the biological response is exhibited after application of the light). In the example where the time of application is one minute or less, biological response can extend for ten minutes or more. When the time is thirty seconds or less, the biological response can extend for two minutes or more. In some instances, the peripheral nerve can be the phrenic nerve and the biological response can be related to movement of the diaphragm innervated by the peripheral nerve. In other instances, the peripheral nerve can be the hypoglossal nerve and the biological response can be related to movement of the tongue innervated by the hypoglossal nerve. In still other instances, the peripheral nerve can be an intercostal nerve, and the biological response can be related to movement of a muscle innervated by the intercostal nerve. In further instances, the peripheral nerve can be a spinal motor neuron, and the biological function can be related to movement of a muscle innervated by the spinal motor neuron.

### VII. Experimental

The following experiment shows that neuromodulation of the phrenic nerve of a Sprague-Dawley rat with infrared (IR) light increases phrenic nerve amplitude for an extended period of time without affecting breathing frequency. The following experimental results are shown for the purpose of illustration only and are not intended to limit the scope of the appended claims.

### Experimental Methods

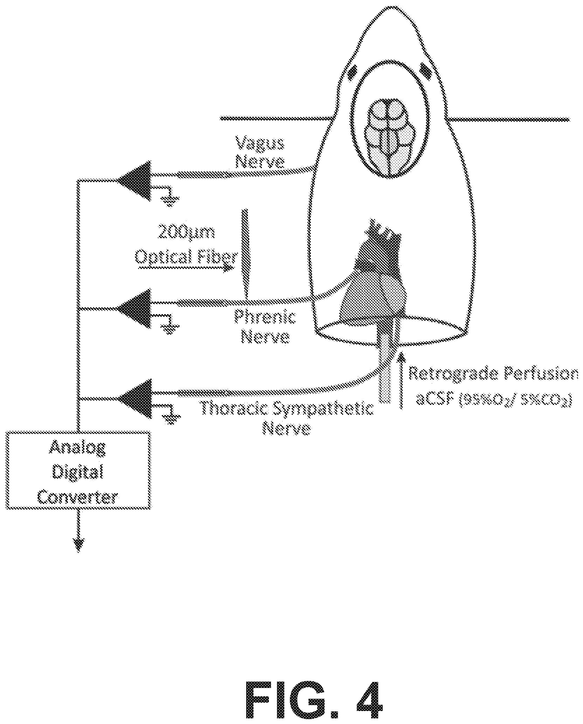

The experimental setup for an *in situ* preparation of rat pups (P15 - P25) is shown in FIG. 4. Vagal (VNA), Phrenic (PNA), and thoracic Sympathetic (tSNA) nerve activities were recorded. Perfusion pressure (PP) was also recorded. The IR diode laser used provided IR light with wavelengths of 1464 nm and 1860 nm, a pulse duration of 200 ms, and a pulse frequency of 200 Hz. The IR diode laser sent the IR light through an optic fiber with a 200 µm diameter.

The protocol for IR control - threshold - included baseline recording (Bsin, 5-10 min), test low level of radiant exposure, progressively increased radiant exposure every 2-5 minutes. The protocol for IR control - long term facilitation - included baseline recording (Bsin, 5-10 min), INC train duration 20 s, 30-40 min post-INC, and repeat.

### Results

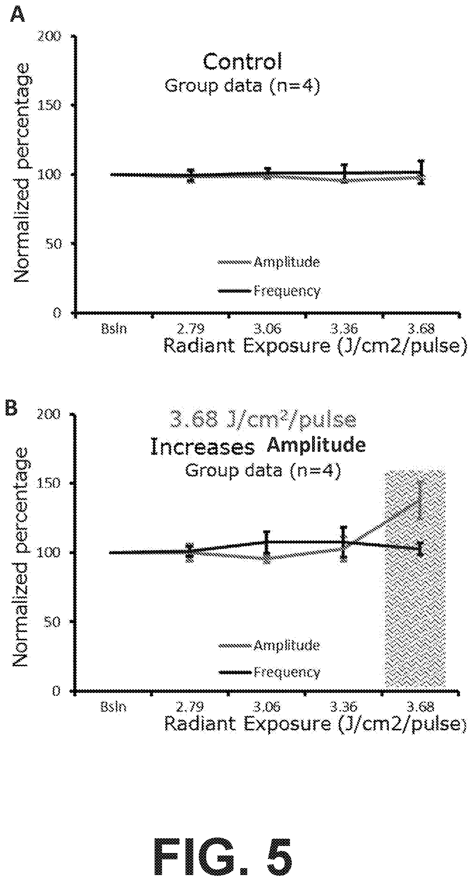

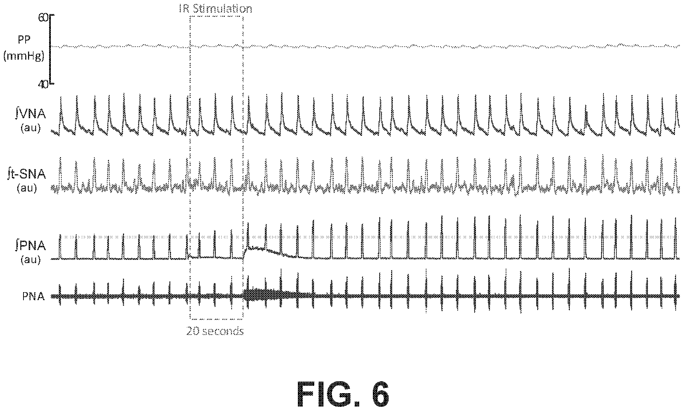

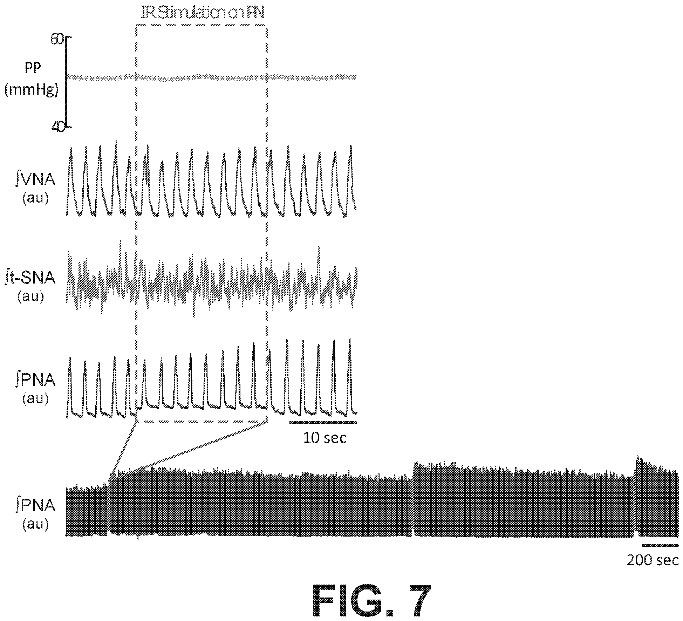

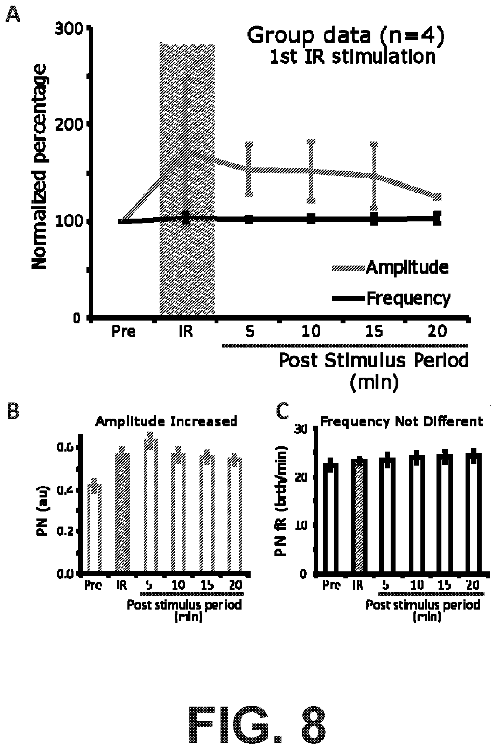

As shown in FIG. 5, IR light increases the amplitude of the phrenic nerve. The increase in amplitude is caused by IR light having a radiant exposure of 0.368 J/cm²/pulse. The frequency is not affected. FIG. 6 illustrates how effective IR light increases the amplitude within the phrenic nerve (PN) without affecting breathing. FIGS. 7 and 8 show how brief IR stimulation evokes long term effects.

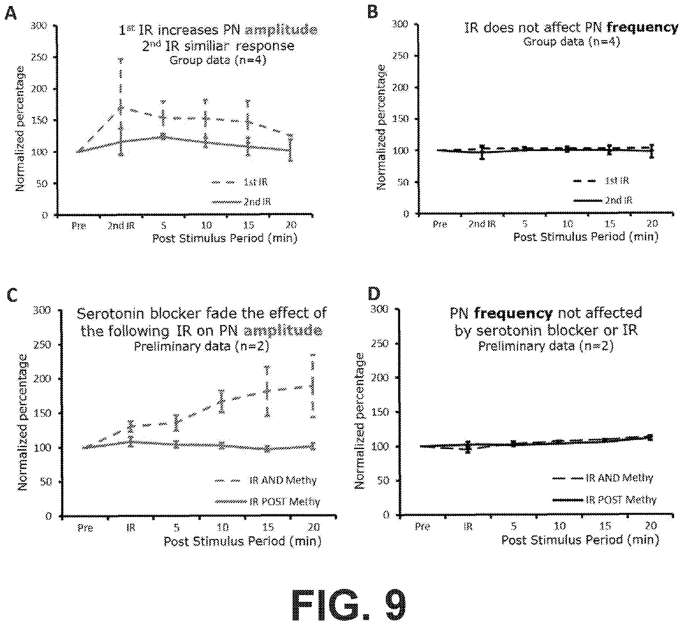

Intermittent hypoxia (IH) works via serotonin to increase the amplitude of the phrenic nerve and other non-respiratory motor neurons. IR may work through the same mechanism as IH. Activation of sensory afferents - TRPV channels - serotonin pathway induction - enhancement of motor activity of the phrenic nerve. As shown in FIG. 9, application of a serotonin blocker (Methysergide via microinjection in the phrenic motor nucleus at the level of C5) diminished the effect of subsequent IR application (and phrenic long term facilitation of the subsequent IR stimulation).

From the above description, those skilled in the art will perceive improvements, changes and modifications. Such improvements, changes and modifications are within the skill of one in the art and are intended to be covered by the appended claims, which define the present invention.

## Claims

1. A system (10) comprising:
a controller (12) configured to configure a light signal with an intensity and a time of application to achieve a target biological effect, wherein the time of application is thirty seconds or less and the intensity is an amount of radiant exposure to be delivered during the time of application;
a generator (14) configured to receive the configuration from the controller and generate the light signal according to the intensity and the time of application; and
an optrode (16) configured to be placed proximal to at least a portion of a peripheral nerve to apply the light signal to the portion of the peripheral nerve to deliver the amount of radiant exposure during the time to at least one axon within the portion of the peripheral nerve to achieve the target biological effect, wherein the at least one axon is configured to activate at least one muscle associated with the target biological effect to achieve the target biological effect,
wherein the target biological effect extends for at least two minutes beyond the time of application.

2. The system of claim 1, wherein the optrode is within a cuff wrapped around at least the portion of the peripheral nerve.

3. The system of claim 1 or claim 2, wherein the light signal is an infrared (IR) light signal and the optrode comprises at least one of an IR light emitting diode and an IR laser.

4. The system of any preceding claim, wherein the optrode is connected to the light generator by a flexible fiber optic cable to transmit the light signal between the light generator and the optrode,
wherein the flexible fiber optic cable comprises a polymer material.

5. The system of any preceding claim, wherein the target biological effect is related to movement of the at least one muscle.

6. The system of any preceding claim, wherein the application of light to the portion of the peripheral nerve increases an amplitude of conduction within the selected at least one axon.

## Patentansprüche

1. System (10) umfassend:
eine Steuervorrichtung (12), die ausgebildet ist, um ein Lichtsignal mit einer Intensität und einer Anwendungszeit zu bilden, um einen gezielten biologischen Effekt zu erreichen, wobei die Anwendungszeit dreißig Sekunden oder weniger beträgt und die Intensität ein Maß an Strahlungseinwirkung ist, die während der Anwendungszeit abgegeben werden soll;
einen Generator (14), der ausgebildet ist, die Konfiguration von der Steuervorrichtung zu empfangen und das Lichtsignal in Abhängigkeit von der Intensität und der Anwendungszeit zu erzeugen; und
eine Optrode (16), die ausgebildet ist, um proximal zu mindestens einem Teil eines peripheren Nervs platziert zu werden, um das Lichtsignal auf den Teil des peripheren Nervs anzuwenden, um während der Zeit das Maß an Strahlungseinwirkung an mindestens ein Axon innerhalb des Teils des peripheren Nervs zu liefern, um den gezielten biologischen Effekt zu erreichen, wobei das mindestens eine Axon ausgebildet ist, um mindestens einen mit dem gezielten biologischen Effekt verbundenen Muskel zu aktivieren, um den gezielten biologischen Effekt zu erreichen,
wobei der gezielte biologische Effekt für mindestens zwei Minuten über den Zeitpunkt der Anwendung hinaus anhält.

2. System nach Anspruch 1, wobei sich die Optrode innerhalb einer Manschette befindet, die um mindestens den Teil des peripheren Nervs gewickelt ist.

3. System nach Anspruch 1 oder Anspruch 2, wobei das Lichtsignal ein Infrarot (IR)-Lichtsignal ist und die Optrode mindestens eine IR-Licht emittierende Diode oder einen IR-Laser aufweist.

4. System nach einem der vorhergehenden Ansprüche, wobei die Optrode mit dem Lichtgenerator durch ein flexibles Glasfaserkabel verbunden ist, um das Lichtsignal zwischen dem Lichtgenerator und der Optrode zu übertragen, wobei das flexible Glasfaserkabel ein Polymermaterial aufweist.

5. System nach einem der vorhergehenden Ansprüche, wobei der gezielte biologische Effekt mit der Bewegung des mindestens einen Muskels verbunden ist.

6. System nach einem der vorhergehenden Ansprüche, wobei die Anwendung von Licht auf den Teil des peripheren Nervs eine Amplitude der Leitung innerhalb des ausgewählten mindestens einen Axons erhöht.

## Revendications

1. Système (10) comprenant
un contrôleur (12) configuré pour configurer un signal lumineux avec une intensité et un temps d'application pour obtenir un effet biologique visé, le temps d'application étant de trente secondes ou moins et l'intensité étant une quantité d'exposition radiométrique à fournir pendant le temps d'application,
un générateur configuré pour recevoir la configuration du contrôleur et générer le signal lumineux correspondant à l'intensité et au temps d'application et
une optrode (16) configurée pour être placée à proximité d'au moins une partie d'un nerf périphérique pour appliquer le signal lumineux à la partie du nerf périphérique afin de fournir la quantité d'exposition radiométrique pendant le laps de temps à au moins un axone dans la partie du nerf périphérique pour obtenir l'effet biologique visé, lequel au moins un axone est configuré pour activer au moins un muscle associé à l'effet biologique visé pour obtenir l'effet biologique visé,
lequel effet biologique visé s'étend pendant au moins deux minutes au-delà du temps d'application.

2. Système selon la revendication 1, dans lequel l'optrode est dans une manchette enroulée autour au moins de la partie du nerf périphérique.

3. Système selon la revendication 1 ou la revendication 2, dans lequel le signal lumineux est un signal lumineux infrarouge (IR) et l'optrode comprend au moins un élément parmi une diode lumineuse IR et un laser IR.

4. Système selon l'une quelconque des revendications précédentes, dans lequel l'optrode est connectée au générateur de lumière par un câble de fibre optique flexible pour transmettre le signal lumineux entre le générateur de lumière et l'optrode, lequel câble de fibre optique flexible comprend un matériau polymère.

5. Système selon l'une quelconque des revendications précédentes, dans lequel l'effet biologique visé est lié au mouvement de l'au moins un muscle.

6. Système selon l'une quelconque des revendications précédentes, dans lequel l'application de lumière à la partie du nerf périphérique augmente une amplitude de conduction à l'intérieur de l'au moins un axone sélectionné.
